(19) Europäisches Patentamt
European Patent Office
Office européen des brevets

(11) Veröffentlichungsnummer: **0 202 553**
**B1**

# (12) EUROPÄISCHE PATENTSCHRIFT

(45) Veröffentlichungstag der Patentschrift: **02.08.89**

(51) Int. Cl.⁴: **C07C 49/303**, C07C 31/135, C07C 69/02, C07C 69/14, C07C 43/04, A23L 1/226, C11B 9/00, A61K 7/46

(21) Anmeldenummer: **86106314.7**

(22) Anmeldetag: **09.05.86**

## (54) Cyclohexanderivate.

(30) Priorität: **11.05.85 DE 3517106**

(43) Veröffentlichungstag der Anmeldung: **26.11.86 Patentblatt 86/48**

(45) Bekanntmachung des Hinweises auf die Patenterteilung: **02.08.89 Patentblatt 89/31**

(84) Benannte Vertragsstaaten: **BE CH DE FR GB IT LI NL**

(56) Entgegenhaltungen:
**FR-A- 2 335 244**
**FR-A- 2 374 046**
**GB-A- 1 159 188**
**GB-A- 2 000 967**

(73) Patentinhaber: **BASF Aktiengesellschaft, Carl-Bosch-Strasse 38, D-6700 Ludwigshafen(DE)**

(72) Erfinder: **Gramlich, Walter, Dr., Auf der Hoehe 11, D-6803 Edingen-Neckarhausen(DE)**
Erfinder: **Schuster, Ludwig, Dr., Weinheimer Strasse 44, D-6703 Limburgerhof(DE)**

**EP 0 202 553 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindug betrifft neue Cyclohexanderivate der allgemeinen Formel I

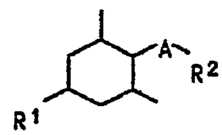

I

in der $R^1$ und $R^2$ für $C_1$-$C_5$-Alkylreste stehen und A eine Karbonylgruppe, eine Hydroxymethylengruppe, eine $C_1$-$C_5$-Alkoxymethylengruppe oder eine $C_1$-$C_6$- Acyloxymethylengruppe bedeutet.

Außerdem betrifft die Erfindung, entsprechend der Aufgabe, neue Duft- und Aromastoffe mit eigentümlicher Charakteristik bereitzustellen, die Verwendung der Cyclohexanderivate I als Duft- und Aromastoffe für Lebensmittel, kosmetische Zubereitungen und Reinigungs- und Pflegemittel des Haushalts.

Je nach der Bedeutung der Gruppierung A handelt es sich im einzelnen um die Cyclohexanderivate Ia-d

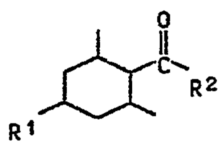

Ia

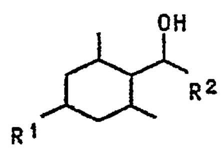

Ib

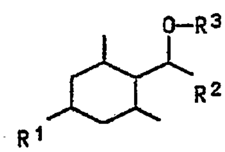

Id

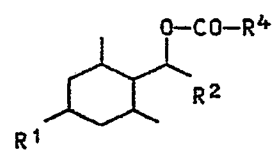

Id

wobei $R^3$ und $R^4$ definitionsgemäß $C_1$-$C_5$-Alkylgruppen und $R^4$ darüberhinaus Wasserstoff bedeuten.

Der Rest $R^1$ steht in allen Fällen in erster Linie für die Methylgruppe und die tert.-Butylgruppe sowie des weiteren für die Ethyl- und Isopropylgruppe.

Bevorzugte Reste $R^2$ sind, ebenfalls in allen Fällen, die Methylgruppe sowie daneben n-Alkylreste wie die n-Propylgruppe. Unter den verzweigten Resten $R^2$ ist die Isobutylgruppe hervorzuheben. Als Reste $R^3$ und $R^4$ kommen vor allem die Methyl- und die Ethylgruppe in Betracht.

Als wichtige Beispiele seien genannt
- für Verbindungen des Typs Ia:
1-(2,4,6-Trimethyl-cyclohex-1-yl)-methyl-keton
1-(2,4,6-Trimethyl-cyclohex-1-yl)-propyl-keton
- für Verbindungen des Typs Ib:
1-(2,4,6-Trimethyl-cyclohex-1-yl)-ethan-1-ol
1-(2,6-Dimethyl-4-tert.butyl-cyclohex-1-yl)-ethan-1-ol
1-(2,4,6-Trimethyl-cyclohex-1-yl)-butan-1-ol
- für Verbindungen des Typs Ic:
1-(2,4,6-Trimethyl-cyclohex-1-yl)-1-methoxyethan, und
- für Verbindungen des Typs Id:
1-(2,4,6-Trimethyl-cyclohex-1-yl)-1-acetoxyethan.

Die Verbindungen I sind in an sich bekannter Weise aus den entsprechenden Phenyl-alkylketonen II

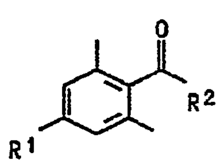

II

erhältlich, die man ihrerseits zweckmäßigerweise durch Acylierung der 3,5-Dimethylalkylbenzole III

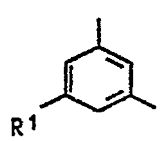

III

mit einem Säurechlorid IV

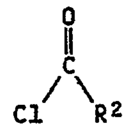

IV

nach Friedel-Crafts herstellt.

Unterwirft man II einer Perhydrierung, z.B. mit Wasserstoff und Raney-Nickel, Palladium oder Rhodium bei 150 - 250°C und 50 - 200 bar $H_2$-Druck, so erhält man die Alkohole Ib, die man in gewohnter Weise zur Herstellung der Ethere Ic, z.B. mit Alkylhalogeniden $R^3$-Hal oder Dialkylsulfaten $(R^3\text{-}O)_2SO_2$ verethern oder zur Herstellung der Ester Id mit einer Säure $R^4$-COOH wie üblich verestern kann.

Ferner kann man die Oxogruppe von II unter milden Bedingungen auch zunächst partiell hydrieren, und die Kernhydrierung, gegebenenfalls nach Veresterung des entstehenden 1-Phenylalkanols hieran anschließen, wobei die gleichen Katalysatoren wie für die Perhydrierung in Betracht kommen.

Die Verbindungen Ia sind neben den Verbindungen Ib durch partielle Kernhydrierung von II mit den genannten Katalysatoren, jedoch unter milderen Bedinungen (100 - 150°C; 30 - 150 bar; geringere Konzentration von II in dem Hydrierungsgemisch) in etwa bis zu 40%iger Ausbeute) erhältlich. Legt man auf die alleinige Synthese Ia Wert, empfiehlt es sich jedoch, Ia durch Oxidation von Ib, beispielsweise mit Natriumdichromat oder Chromsäure, herzustellen.

Die Verbindungen Ib-d fallen bei den hier skizzierten Herstellungsverfahren als Racemate (mit dem C-Atom der Alkoholfunktion als chiralem Zentrum) an und können gewünschtenfalls nach den üblichen Methoden der Racematspaltung in ihre optischen Antipoden getrennt werden. Nach den bisherigen Beobachtungen unterscheiden sich die reinen Enantiomeren hinsichtlich ihrer olfaktorischen Eigenschaften nur unwesentlich von den Racematen, so daß die Darstellung der optisch aktiven Verbindungen im allgemeinen entbehrlich ist.

Die Cyclohexanderivate I weisen allgemein einen würzig-krautigen bis blumigen Geruch auf und zeichnen sich in den einzelnen Fällen durch ausgeprägte individuelle Noten aus. Sie stellen daher eine wesentliche Bereicherung der Duft- und Aromastoffe dar und eignen sich insbesondere sowohl für die Aromatisierung von Lebensmitteln als auch zur Herstellung von Parfümen, die entweder als solche Verwendung finden oder zur Parfümierung von sonstigen kosmetischen Zubereitungen aller Art oder für Reinigungs- und Pflegemittel des Haushalts dienen.

Beispiel 1

1-(2,4,6-Trimethyl-cyclohex-1-yl)-ethan-1-ol (Ib/1)

Eine Mischung aus 400 g 2,4,6-Trimethylacetophenon (Acetylmesitylen), 1 g Rutheniumhydroxid und 50 ml Tetrahydrofuran wurde 5 h bei 180°C und einem $H_2$-Druck von 200 bar der Perhydrierung unterworfen, wonach das Gemisch wie üblich auf die obengenannte Verbindung aufgearbeitet wurde; Ausbeute 90 %; Kp. 114°C/20 mbar.

Geruch: minzig-erdig mit pfeffriger Note

Beispiel 2

1-(2,4,6-Trimethyl-cyclohex-1-yl)-1-acetoxyethan (Id/1)

207 g Ib/1 (s. Beispiel 1) wurde mit 306 g Acetanhydrid 5 h unter Rückflußkühlung erhitzt und danach wie üblich auf die obengenannte Verbindung aufgearbeitet; Ausbeute 92 %; Kp. 78°C/0,005 mbar.

Geruch: holzig-pudrig mit Ionon-Note

Beispiel 3

1-(2,4,6-Trimethyl-cyclohex-1-yl)-methyl-keton (Ia/1)

Eine Mischung aus 100 g Acetylmesitylen, 1 g Rutheniumhydroxid und 500 ml Tetrahydrofuran wurde 5 h bei 120°C und bei einem $H_2$-Druck von 50 bar der Hydrierung unterworfen. Die übliche Aufarbeitung des Gemisches lieferte neben 60 % des perhydrierten Produktes Ib/1 31 % der obengenannten Verbindung; Kp. 98°C/20 mbar.

Geruch: krautig, mit holziger Note

Beispiel 4

1-(2,4,6-Trimethyl-cyclohex-1-yl)-butan-1-ol (Id/2)

Eine Mischung aus 1200 g Mesitylen und 800 g $AlCl_3$ wurde bei 60°C im Laufe von 2 h mit 426 g Butyroylchlorid versetzt und nach 12stündigem Rühren bei Raumtemperatur auf das 1-(2,4,6-Trimethyl-phen-1-yl)-propyl-keton aufgearbeitet; Ausbeute 62,5 %; Kp. 79°C/0,05 mbar.

Dieses Keton wurde dann analog Beispiel 1 zu der obengenannten Verbindung hydriert; Ausbeute, bezogen auf das Keton, 88 %; Kp. 64°C/0,01 mbar.

Geruch: krautig, mit Estragon/Muskat-Note

Beispiel 5

1-(2,4,6-Trimethyl-cyclohex-1-yl)-propyl-keton (Ia/2)

Die Hydrierung von 1-(2,4,6-Trimethyl-phen-1-yl)-propyl-keton (s. Beispiel 4) unter den Bedingungen von Beispiel 3 lieferte neben 61 % des perhydrierten Produktes Ib/2 32 % der obengenannten Verbindung; Kp. 59°C/0,1 mbar.

Geruch: krautig, mit Anis/Fenchel-Note

Beispiel 6

1-(2,6-Dimethyl-4-tert.-butyl-cyclohex-1-yl)-ethan-1-ol (Ib/3)

Auf die in Beispiel 1 angegebene Weise wurden 400 g 1-(2,6-Dimethyl-4-tert.-butyl-phen-1-yl)-methylketon in die obengenannte Verbindung überführt; Ausbeute 89 %; Kp. 84°C/0,2 mbar.

Geruch: holzig-erdig mit Vetiver-Note

Beispiel 7

Ein bekanntes Eau de Toilette (W.A.Bucher, "Perfumes, Cosmetics and Soaps", Vol. VII, Seite 264, Chapman and Hall, London, 1974) vom Lavendel-Typ der Zusammensetzung

20 g Lavendelöl (französisch)
10 g Bergamottöl
2 g Lemongrasöl
3 g Muskateller-Salbeiöl
1 g Ambrette-Moschus
5 g Castoreum-Extrakt (3 gew.%ig)
25 g Ambergris Essence (3 gew.%ig)
1000 g Ethanol (80Vol.%)

wurde durch Zusatz von 6 g Ib/2 (s. Beispiel 4) abgewandelt, wodurch dessen frischer Charakter angenehm ins Würzige verstärkt wurde.

## Patentansprüche

1. Cyclohexanderivate der allgemeinen Formel I

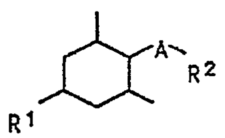

I

in der $R^1$ und $R^2$ für $C_1$-$C_5$-Alkylreste stehen und A eine Karbonylgruppe, eine Hydroxymethylengruppe, eine $C_1$-$C_5$-Alkoxymethylengruppe oder eine $C_1$-$C_6$-Acyloxymethylengruppe bedeutet.

2. 1-(2,4,6-Trimethyl-cyclohex-1-yl)-methyl-keton
3. 1-(2,4,6-Trimethyl-cyclohex-1-yl)-propyl-keton
4. 1-(2,4,6-Trimethyl-cyclohex-1-yl)-ethan-1-ol
5. 1-(2,6-Dimethyl-4-tert.butyl-cyclohex-1-yl)-ethan-1-ol
6. 1-(2,4,6-Trimethyl-cyclohex-1-yl)-butan-1-ol
7. 1-(2,4,6-Trimethyl-cyclohex-1-yl)-1-acetoxyethan
8. Verwendung der Cyclohexanderivate I als Duft-und Aromastoffe für Lebensmittel, kosmetische Zubereitungen und Reinigungs- und Pflegemittel für den Haushalt.
9. Lebensmittel, kosmetische Zubereitungen und Reinigungs- und Pflegemittel für den Haushalt, enthaltend die Cyclohexanderivate I als Duft- und Aromastoffe.

## Claims

1. A cyclohexane derivative of the formula I

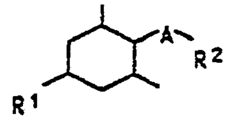

I

where $R^1$ and $R^2$ are each $C_1$–$C_5$-alkyl and A is carbonyl, hydroxymethylene, $C_1$–$C_5$-alkoxymethylene or $C_1$–$C_6$-acyloxymethylene.

2. 2,4,6-Trimethylcyclohex-1-yl methyl ketone.
3. 2,4 6-Trimethylcyclohex-1-yl propyl ketone.

4. 1-(2,4,6-Trimethylcyclohex-1-yl)-ethan-1-ol.
5. 1-(2,6-Dimethyl-4-tert-butylcyclohex-1-yl)-ethan-1-ol.
6. 1-(2,4,6-Trimethylcyclohex-1-yl)-butan-1-ol.
7. 1-(2,4,6-Trimethylcyclohex-1-yl)-1-acetoxyethane.
8. Use of the cyclohexane derivative I as a fragrance or aroma for foods, cosmetic formulations and household cleaners and polishes.
9. A foodstuff, a cosmetic formulation of a household cleaner or polish containing the cyclohexane derivative I as a fragrance or aroma.

## Revendications

1. Dérivés de cyclohexane de formule générale I

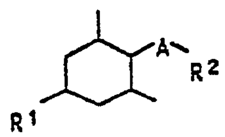

I

dans laquelle $R^1$ et $R^2$ représentent des restes alkyle en $C_1$–$C_5$ et A un groupement carbonyle, un groupement hydroxyméthylène, un groupement (alcoxy en $C_1$–$C_5$)-méthylène ou un groupement (acyloxy en $C_1$-$C_5$)méthylène.
2. 1-(2,4,6-Triméthyl-cyclohex-1-yl)-méthyl-cétone.
3. 1-(2,4,6-Triméthyl-cyclohex-1-yl)-propyl-cétone.
4. 1-(2,4,6-Triméthyl-cyclohex-1-yl)-éthan-1-ol.
5. 1-(2,6-Diméthyl-4-tert.butyl-cyclohex-1-yl)-éthan-1-ol.
6. 1-(2,4,6-Triméthyl-cyclohex-1-yl)-butan-1-ol.
7. 1-(2,4,6-Triméthyl-cyclohex-1-yl)-1-acétoxyéthane.
8. Utilisation des dérivés de cyclohexane I comme parfums et aromatisants pour aliments, préparations cosmétiques et produits de nettoyage et d'entretien à usage ménager.
9. Aliments, préparations cosmétiques et produits de nettoyage et d'entretien à usage ménager, contenant les dérivés de cyclohexane I comme parfums et aromatisants.